# EUROPEAN PATENT SPECIFICATION

(11) **EP 1 452 186 B1**
(45) Date of publication and mention of the grant of the patent: **09.02.2011**
(21) Application number: 02791962.0
(22) Date of filing: 05.12.2002
(51) Int. Cl.: A61L 2/06, A61B 1/00, A61L 2/07

(54) **HIGH-PRESSURE STEAM STERILIZATION SYSTEM FOR MEDICAL EQUIPMENT, AND DEVICE AND METHOD FOR STERILIZING THE MEDICAL EQUIPMENT**
HOCHDRUCK-DAMPFSTERILISATIONSSYSTEM FÜR MEDIZINISCHE GERÄTE UND VORRICHTUNG UND VERFAHREN ZUR STERILISIERUNG DER MEDIZINISCHEN GERÄTE
SYSTEME DE STERILISATION A LA VAPEUR HAUTE PRESSION POUR EQUIPEMENT MEDICAL ET DISPOSITIF ET PROCEDE DE STERILISATION DE CET EQUIPEMENT

(30) Priority: 07.12.2001 JP 2001374703
(43) Date of publication of application: 01.09.2004
(73) Proprietor: Olympus Corporation, Shibuya-ku, Tokyo (JP)
(72) Inventor: ISHIBIKI, Kota, Hachioji-shi, Tokyo 192-0916 (JP)
(74) Representative: von Hellfeld, Axel
(86) International application number: PCT/JP2002/012757
(87) International publication number: WO 2003/047638

(56) References cited:
- WO-A-01/76646
- FR-A- 2 774 913
- GB-A- 1 570 096
- JP-A- 5 285 103
- JP-A- 5 337 082
- JP-A- 2000 024 094
- JP-A- 2000 051 323
- JP-A- 2000 225 179
- JP-A- 2001 224 666
- JP-A- 2002 253 477
- JP-A- 2002 263 172
- JP-U- 4 128 747
- US-A- 4 335 071
- US-A1- 2002 001 551

## Description

### Technical Field

The present invention relates to a medical equipment autoclaving system, a medical equipment autoclave, and a medical equipment autoclaving method for autoclaving medical equipment that has a soft armor member.

### Background Art

In recent years, endoscopes of which an insertion unit is inserted into a body cavity for the purpose of observation of a deep region in the body cavity or which permit cures and treatments using, if necessary, a treatment instrument in combination have been widely adopted in the field of medicine. For the endoscope that is medical equipment, it is indispensable to reliably disinfect or sterilize a used endoscope for the purpose of preventing infectious diseases or the like.

Conventional disinfection and sterilization depends on a gas such as ethylene oxide or a disinfectant and requires time-consuming work. Moreover, the disinfection and sterilization requires much time for aeration that is performed in order to remove the gas adhering to equipment after completion of sterilization. This poses a problem in that the equipment cannot be used immediately after the completion of sterilization. Furthermore, the disinfection and sterilization requires a high running cost.

Autoclaving that does not require the time-consuming work, permits use of an endoscope immediately after completion of autoclaving, and requires only a low running cost is becoming the mainstream of sterilization of medical equipment.

Typical conditions for autoclaving are stipulated in the ANSI/AAMI ST37-1992 issued from the American National Standards Institute and the Association for the Advancement of Medical instrumentation. The conditions are such that a pre-vacuum type sterilization process should be performed at 132°C for 4 min., and a gravity-type sterilization process should be performed at 132°C for 10 min. The temperature for autoclaving often ranges from 115°C to 140°C.

A general pre-vacuum type autoclaving process comprises a pre-vacuum process for depressurizing (applying negative pressure to) a sterilization chamber in which medical equipment that is an object of sterilization is stored, a sterilization process for sterilizing the medical equipment by feeding high-pressure high-temperature steam to the sterilization chamber, and a dry process for after the completion of the sterilization, depressurizing the sterilization chamber for the purpose of drying an endoscope.

The pre-vacuum process is a process intended to facilitate infiltration of steam into every corner of the medical equipment at the succeeding sterilization process. The sterilization chamber is depressurized, whereby the high-pressure high-temperature steam permeates the entire medical equipment stored. The pre-vacuum process is adopted for gas sterilization that utilizes an ethylene oxide gas.

The pressure in the sterilization chamber in the pre-vacuum process and dry process alike is about -0.07 MPa with respect to the atmospheric pressure. On the other hand, the pressure in the sterilization process is often set to +0.2 MPa with respect to the atmospheric pressure.

In general, when the insertion unit of an endoscope is soft or the insertion unit of an endoscope includes a bending section, an armor tube made of a soft high polymer material such as a rubber or elastomer is adopted as an armor member that sheathes the soft insertion unit or bending section. Moreover, the endoscope has a watertight sealable structure so that it can be immersed in a liquid agent.

Consequently, when an endoscope is autoclaved while being kept watertight, the armor tube that is the softest among all the casing members of the endoscope is likely to expand during depressurization performed in the pre-vacuum process or the like. There is a fear that the durability of the endoscope may be impaired. Likewise, a relatively feeble portion of a joint between parts of the endoscope may be affected by a difference in pressure between the inside and outside of the endoscope. Consequently, the durability of the endoscope may be degraded.

Japanese Unexamined Patent Application Publication No. 2001-70226 discloses an art of attaching a check valve cap to a camera connector 7 that is a joint between an endoscope and peripheral equipment prior to autoclaving. In the related art, when the internal pressure of an endoscope relatively rises during a depressurization process preceding autoclaving, a check valve included in the check valve cap is opened in order to prevent the relative rise of the internal pressure. Moreover, autoclaving is performed under the pressure higher than the internal pressure of the endoscope. Therefore, the check valve is closed to hinder active invasion of high-pressure high-temperature steam.

However, as far as the endoscope described in the Japanese Unexamined Patent Application Publication No. 2001-70226 is concerned, the pressure resulting from depressurization performed in the pre-vacuum process preceding autoclaving is nearly the same as pressure resulting from depressurization performed in the dry process succeeding the autoclaving process. Consequently, the internal pressure of the endoscope attained after the completion of the autoclaving is held at the pressure resulting from the depressurization performed in the pre-vacuum process. During the depressurization in the dry process, a difference in pressure hardly takes place between the inside of the endoscope and the outside thereof. The check valve of the endoscope does not therefore open. Otherwise, if the check valve opens, it opens instantaneously. Therefore, the inside of the endoscope cannot be dried. Consequently, moisture having infiltrated through an armor resin sheathing an insertion unit or O rings is accumulated to deteriorate the internal parts of the endoscope.

For example, assuming that moisture stays near an electric contact via which an endoscope is connected to an external unit such as an image processing unit, when the endoscope is connected to the external unit, contact pins may be short-circuited. Consequently, the endoscope and external unit may be electrically broken.

Accordingly, an object of the present invention is to provide a medical equipment autoclaving system and a medical equipment autoclaving method capable of avoiding drawbacks including deterioration of the internal parts of medical equipment deriving from humidity while achieving autoclaving.

### Disclosure of Invention

Some or all of the above problems are overcome by a medical equipment autoclaving system having the features of claim 1 and a medical equipment autoclaving method having the features of claim 6.

A medical equipment autoclaving system in accordance with the present invention comprises: a communication vent through which the inside of medical equipment and the outside thereof communicate with each other; a pressure adjusting means having a check valve that opens only when the pressure in the inside of the medical equipment which communicates with the outside thereof through the communication vent gets higher by a certain value or more than the pressure in the outside thereof; and an autoclave that sterilizes the medical equipment. The autoclave is designed to execute:
a first depressurization process including a step of depressurizing a chamber included in the autoclave;
an autoclaving process which succeeds the first depressurization process and in which the chamber is pressurized; and
a second depressurization process succeeding the autoclaving process and including a step of depressurizing the chamber.

The pressure attained at at least one depressurizing step included in the second depressurization process is lower than the lowest pressure attained in the first depressurization process.

Moreover, a medical equipment autoclaving method in accordance with the present invention comprises:
a first depressurization process including a step of depressurizing a chamber included in an autoclave in which medical equipment is stored;
an autoclaving process which succeeds the first depressurization process and in which the chamber is pressurized; and
a second depressurization process succeeding the autoclaving process and including a step of depressurizing the chamber.

The pressure attained at at least one depressurizing step included in the second depressurization process to be executed by the autoclave is lower than the lowest pressure attained in the first depressurization process.

Moreover, a medical equipment autoclave may comprise: a chamber that can be sealed with medical equipment, which is an object of sterilization, stored therein; a vacuum pump used to depressurize the inside of the chamber; a steam feeder that feeds steam into the chamber; and a control unit that controls the action of the vacuum pump and the action of the steam feeder.

The control unit executes:
a pre-vacuum process for depressurizing the inside of the chamber by activating the vacuum pump;
an autoclaving process in which after the pre-vacuum process is completed by inactivating the vacuum pump, steam is fed to the inside of the chamber by activating the steam feeder, and the inside of the chamber is thus pressurized;
a dry process in which after the autoclaving process is completed by inactivating the steam feeder, the inside of the chamber is depressurized by activating the vacuum pump, and the medical equipment stored in the chamber is thus dried.

Furthermore, the control unit controls the action of the vacuum pump so that the lowest pressure attained at the dry process will be lower than the lowest pressure attained in the pre-vacuum process.

Moreover, a medical equipment autoclaving method may comprise:
a pre-vacuum process for depressurizing the inside of a chamber, in which medical equipment that is an object of sterilization is stored, using a vacuum pump;
an autoclaving process in which after the pre-vacuum step is completed, a steam feeder for feeding steam into the inside of the chamber is activated in order to feed steam into the inside of the chamber, and the inside of the chamber is thus pressurized in order to sterilize the medical equipment; and
a dry process in which after the autoclaving process is completed, the vacuum pump is activated in order to depressurize the inside of the chamber, and the medical equipment stored in the chamber is thus dried.

The action of the vacuum pump is controlled so that the lowest pressure attained at the dry process will be lower than the lowest pressure attained in the pre-vacuum process.

### Brief Description of the Drawings

Fig. 1 is an explanatory diagram showing the overall configuration of an endoscope system to which a first embodiment of the present invention is adapted;
Fig. 2 is a sectional view showing a pressure adjustment member that is included in an endoscope and relates to the first embodiment of the present invention;
Fig. 3 is a sectional view showing an electric connector that is included in the endoscope and relates to the first embodiment of the present invention;
Fig. 4 is a block diagram showing the configuration of an autoclave, which autoclaves an endoscope, in accordance with the first embodiment of the present invention;
Fig. 5 is an explanatory diagram showing a pressure control method implemented in an autoclaving process to be executed by the autoclave in accordance with the first embodiment of the present invention;
Fig. 6 is an explanatory diagram showing the action of the pressure adjustment member relating to the first embodiment of the present invention;
Fig. 7 is an explanatory diagram showing pressurization of the pressure adjustment member through a pressurization base that relates to the first embodiment of the present invention;
Fig. 8 is an explanatory diagram showing a ventilating means for ventilating the inside of an endoscope shown in Fig. 1 to Fig. 7;
Fig. 9 is an explanatory diagram showing a first variant of the ventilating means shown in Fig. 8;
Fig. 10 is an explanatory diagram showing a second variant of the ventilating means shown in Fig. 8;
Fig. 11 is an explanatory diagram showing an endoscope and a ventilating means relating to a second embodiment of the present invention;
Fig. 12 is an explanatory diagram showing an endoscope and a ventilating means relating to a third embodiment of the present invention;
Fig. 13 is an explanatory diagram showing the overall configuration of an endoscope system to which a fourth embodiment of the present invention is adapted;
Fig. 14 is a front view of an electric connector relating to the fourth embodiment of the present invention;
Fig. 15 is a sectional view showing the electric connector relating to the fourth embodiment of the present invention;
Fig. 16 is a flowchart describing a control sequence to be followed by a humidity control unit relating to the fourth embodiment of the present invention;
Fig. 17 is an explanatory diagram showing the overall configuration of an endoscope system to which a fifth embodiment of the present invention is adapted;
Fig. 18 is a flowchart describing a control sequence to be followed by a humidity control unit relating to the fifth embodiment of the present invention; and
Fig. 19 is a sectional view of a major portion of an endoscope relating to a sixth embodiment of the present invention.

### Best Mode for Carrying Out the Invention

Referring to the drawings, embodiments of the present invention will be described below.

### (First Embodiment)

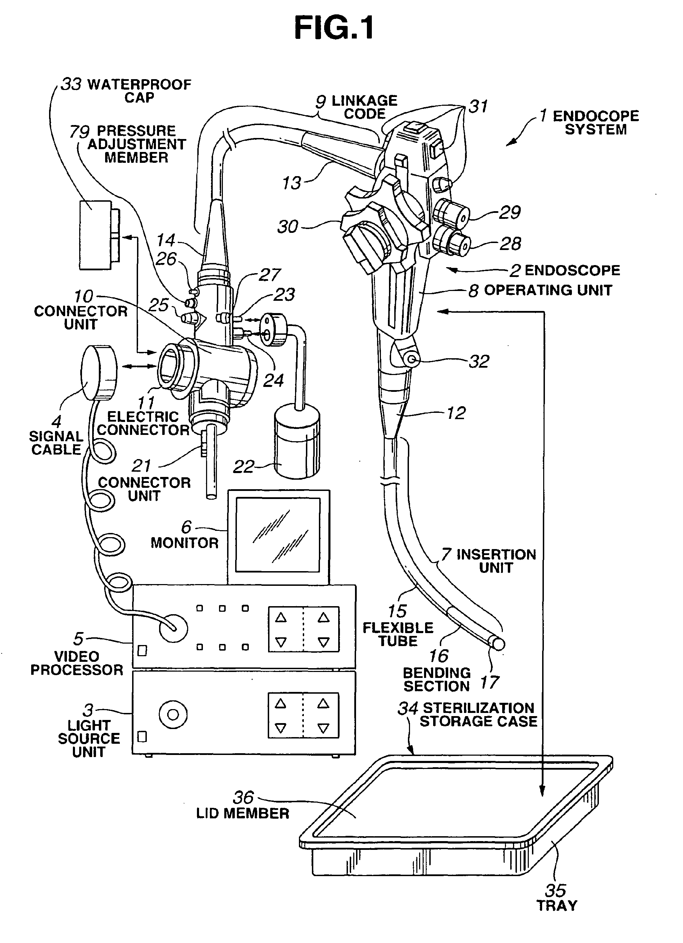

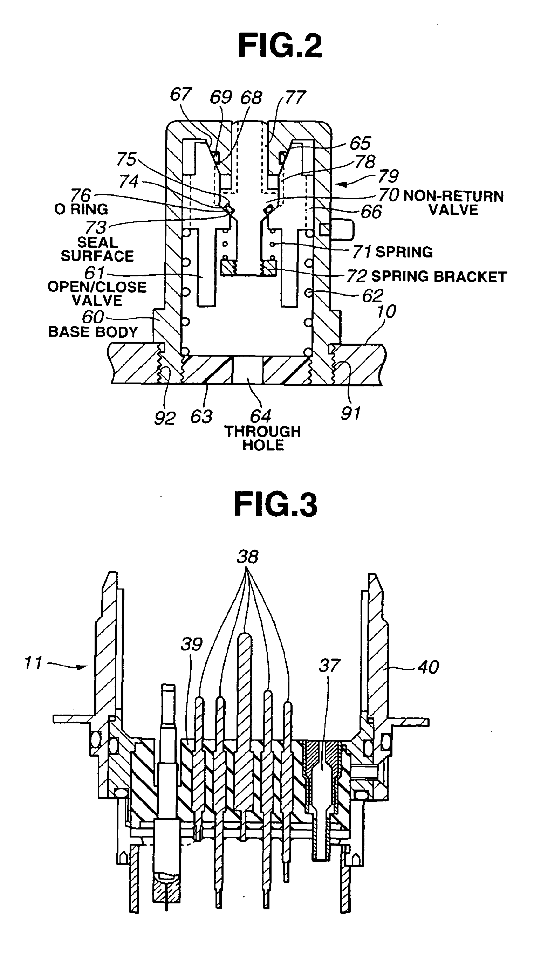

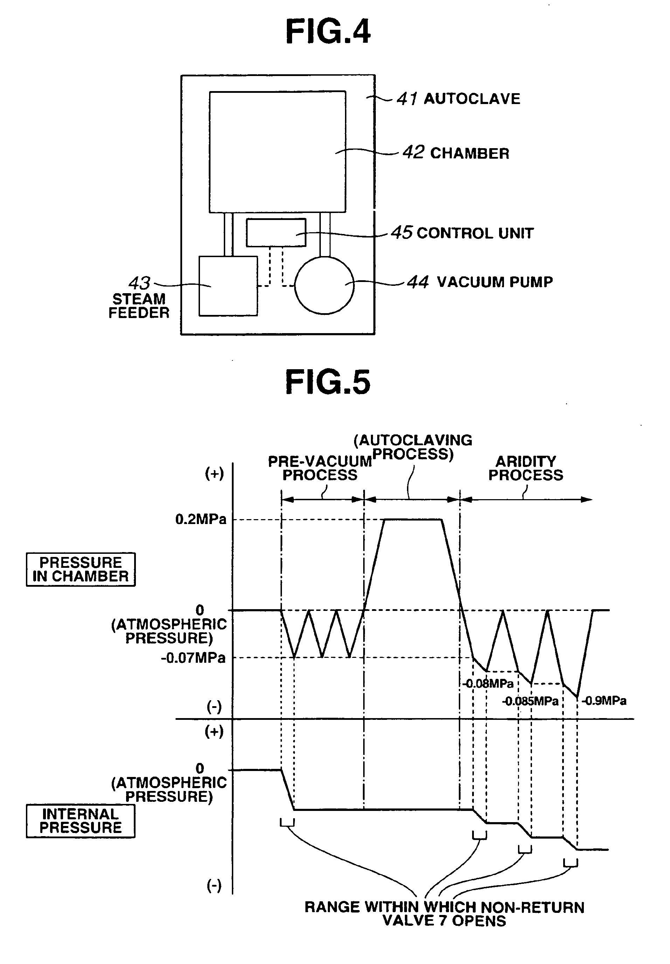

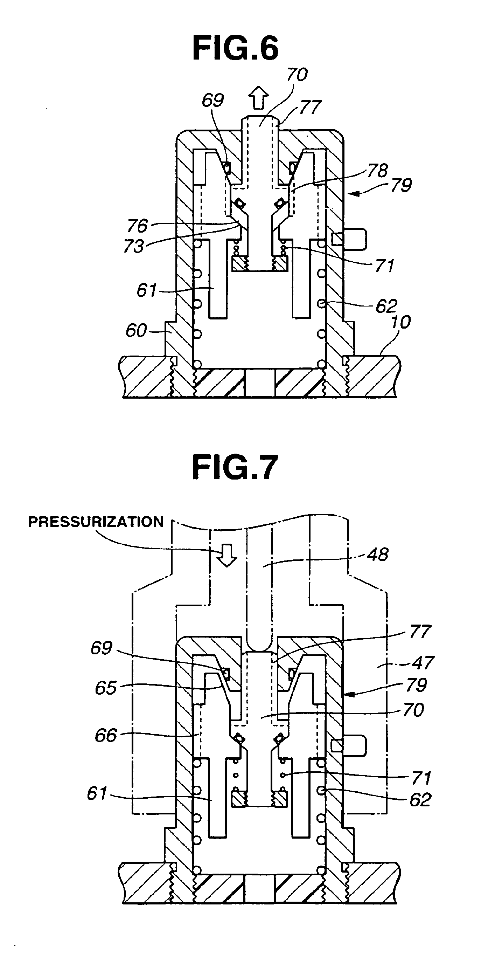

Fig. 1 to Fig. 7 are concerned with a first embodiment of the present invention. Fig. 1 is an explanatory diagram showing the overall configuration of an endoscope system. Fig. 2 is a sectional view showing a pressure adjustment member included in an endoscope. Fig. 3 is a sectional view showing an electric connector included in the endoscope. Fig. 4 is a block diagram showing the configuration of an autoclave that autoclaves the endoscope. Fig. 5 is an explanatory diagram showing a pressure control method to be implemented in an autoclaving process to be executed by the autoclave. Fig. 6 is an explanatory diagram showing the action of the pressure adjustment member. Fig. 7 is an explanatory diagram showing pressurization of the pressure adjustment member through a pressurization base.

### (Constituent Features)

As shown in Fig. 1, an endoscope system 1 comprises an endoscope 2, a light source unit 3, a video processor 5, and a monitor 6. The endoscope 2 has an imaging means. The light source unit 3 is connected to the endoscope 2 so that it can be disconnected freely. The light source unit 3 supplies illumination light to a light guide running through the endoscope 2. The video processor 5 is connected to the endoscope 2 over a signal cable 4, controls the imaging means incorporated in the endoscope 2, and processes a signal sent from the imaging means. A picture corresponding to object images and produced by the video processor 5 is displayed on the monitor 6.

The endoscope 2 has an insertion unit 7, an operating unit 8, a linkage cord 9, a connector unit 10, and an electric connector 11. The insertion unit 7 has flexibility and shaped elongated. The operating unit 8 is coupled to the proximal end of the insertion unit 7. The linkage cord 9 has flexibility and extends from the lateral side of the insertion unit 7. The connector unit 10 is formed at the end of the linkage cord 9 and freely detachably attached to the light source unit 3. The electric connector 11 is formed on the lateral side of the connector unit 10. The signal cable 4 connected to the video processor 5 is freely detachably attached to the electric connector 11.

The electric connector 11 has a vent which will be described later and shown in Fig. 3, and through which the inside of the endoscope 2 and the outside thereof communicate with each other. A joint between the insertion unit 7 and operating unit 8 includes an insertion unit prevention breakage member 12 that includes an elastic member for preventing abrupt bending of the joint. A joint between the operating unit 8 and linkage cord 9 includes an operating unit prevention breakage member 13 similar to the insertion unit prevention breakage member 12. A joint between the linkage cord 9 and connector unit 10 includes a connector unit prevention breakage member 14 similar to the insertion unit prevention breakage member 12.

The insertion unit 7 has a flexible tube 15, a bending section 16, and a distal section 17 concatenated in that order from the proximal end thereof. The flexible tube 15 has flexibility and is soft. The bending section 16 is formed at the distal end of the flexible tube 15, and can be bent by handling the operating unit 8. An aeration/perfusion nozzle through which air or water is supplied, a suction port, a fluid supply port, an observation optical system, and an illumination optical system are included in the distal section 17.

Through the aeration/perfusion nozzle, a cleansing solvent or gas is jetted out towards an optical member located on the outer surface of the observation optical system. The suction port is a distal opening of a treatment instrument channel through which a treatment instrument lying through the insertion unit 7 is projected or through which a humor is sucked from a body cavity. The fluid supply port opens upon an object of observation and is used as an opening through which a fluid is jetted out towards the object of observation.

The connector unit 10 has a gas supply base 21, a water tank pressurization base 23, a fluid supply base 24, a suction base 25, an infusion base 26, and a ground terminal base 27.

A gas source that is not shown and incorporated in the light source unit 3 is coupled to the gas supply base 21 so that it can be uncoupled freely. A water tank 22 serving as a fluid source is coupled to the water tank pressurization base 23 and fluid supply base 24 so that it can be uncoupled freely. A sucker that is not shown and used to suck a fluid through the suction port formed in the distal section 17 is coupled to the suction base 25. A water supply means that is not shown and used to supply water or a fluid through the fluid supply port formed in the distal section 17 is coupled to the infusion base 26. If a high-frequency leakage current flows in the endoscope 2 during diathermy or the like, the leakage current is fed back to a diathermy machine through the ground terminal base 27.

The operating unit 8 has an aeration/perfusion button 28, a suction button 29, an angulation knob 30, a plurality of remote switches 31, and a treatment instrument insertion port 32.

The aeration/perfusion button 28 is an operating member to be handled in order to direct aeration or perfusion. When an operator presses the aeration/perfusion button 28, aeration or perfusion is performed through the aeration/perfusion nozzle formed in the distal section 17. The suction button 29 is an operating member to be handled in order to direct suction. When an operator presses the suction button 29, suction is performed through the suction port formed in the distal section 17.

The angulation knob 30 is an operating member to be handled in order to bend the bending section 16. The plurality of remote switches 31 is operating members to be handled in order to remotely control the video processor 5. The treatment instrument insertion port 32 is the proximal opening of the treatment instrument channel.

Moreover, a waterproof cap 33 can be freely detachably attached to the electric connector 11 of the endoscope 2. The connector unit 10 includes a pressure adjustment member 79.

For autoclaving, a sterilization storage case 34 in which the endoscope 2 is stored is employed. The storage case 34 comprises a tray 35 and a lid member 36. The tray 35 and lid member 36 have a plurality of pores that is not shown. Steam passes through the pores.

Typical conditions for autoclaving are, as mentioned above, stipulated in the ANSI/AAMI ST37-1992 issued from the American National Standards Institute and the Association for the Advancement of Medical instrumentation. The standards stipulate that a pre-vacuum type sterilization process should be executed at 132°C for 4 min, and a gravity-type sterilization process should be executed at 132°C for 10 min.

Next, referring to Fig. 2, the structure of the pressure adjustment member 79 included in the connector unit 10 of the endoscope 2 will be detailed below.

As shown in Fig. 2, the pressure adjustment member 79 is a pressure adjusting means for adjusting the internal pressure of the endoscope 2. The pressure adjustment member 79 is fixed to the connector unit 10 by engaging a screw 92 threaded on a base body 60 with a screw hole 91 threaded on a hole bored in the connector unit 10 of the endoscope 2.

The pressure adjustment member 79 has an opening/closing valve 61 movably put in the base body 60. A spring 62 constrains the opening/closing valve 61 to move upwards in the drawing. A spring bracket 63 prevents the spring 62 from coming off. The spring bracket 63 has a through hole 64 bored therein.

The opening/closing valve 61 has a seal surface 65, a ventilation groove 66, and a ventilation groove 78. An abutment surface 67 that abuts on the seal surface 65 is formed on the base body 60. The abutment surface 67 has an O ring groove 68 formed therein. An O ring 69 is fitted in the O ring groove 68. The O ring 69 seals the seal surface 65 to keep the seal surface 65 watertight.

A check valve 70 is movably engaged with the internal surface of the opening/closing valve 61, and a spring 71 constrains the check valve 70 to move downwards in the drawing. A spring bracket 72 prevents the spring 71 from coming off. The opening/closing valve 61 has a seal surface 73. An abutment surface 74 that abuts on the seal surface 73 is formed on the check valve 70. An O ring groove 75 is formed in the abutment surface 74, and an O ring 76 is fitted in the O ring groove 75. The check valve 70 has a ventilation groove 77. The O ring 76 seals the seal surface 73 to keep the seal surface 73 watertight.

Owing to the foregoing structure, the through hole 64 serves as a communication vent through which the inside and outside of the endoscope 2 communicate with each other. The check valve 70 is a check valve that opens only when the pressure in the inside of the endoscope 2 which communicates with the outside thereof through the communication vent gets higher than the pressure in the outside thereof by a certain value or more.

Next, referring to Fig. 3, the electric connector 11 of the endoscope 2 will be detailed below.

As shown in Fig. 3, the electric connector 11 includes an electric contact through which the endoscope 2 shown in Fig. 1 is connected to a tester for the purpose of testing. Namely, the electric connector 11 has an insulating member 39 that bears electric contact pins 38. A communication vent 37 that is a through hole through which the inside of the endoscope and the outside thereof communicate with each other is bored in the insulating member 39.

An electric connector base 40 of the electric connector 11 is shaped so that the waterproof cap 33 shown in Fig. 1 and designed to block the communication vent 37 in a watertight manner can be freely detachably attached to the electric connector base 40.

Next, Fig. 4 shows the configuration of an autoclave 41 that autoclaves the endoscope 2.

As shown in Fig. 4, the autoclave 41 has a chamber 42, a steam feeder 43, a vacuum pump 44, and a control unit 45. The chamber 42 is a storage means that can be sealed with the endoscope 2 stored therein. The steam feeder 43 is a steam feeding means for feeding steam to the inside of the chamber 42. The vacuum pump 44 is a depressurizing means for depressurizing the inside of the chamber 42. The control unit 45 is a control means such as a CPU included in order to control the actions of the steam feeder 43 and vacuum pump 44 respectively.

The autoclave 41 can execute a first depressurization process that includes a step of depressurizing the chamber 42 included in the autoclave 41, an autoclaving process which succeeds the first depressurization process and in which the chamber 42 is pressurized, and a second depressurization process that succeeds the autoclaving process and includes a step of depressurizing the chamber 42.

Next, referring to Fig. 5, a pressure control method implemented in the autoclaving process to be executed by the autoclave 41 will be described below.

The control unit 45 shown in Fig. 5 is designed to extend control as described below.

As shown in Fig. 5, first, the autoclave 41 executes the first depressurization process generally called a pre-vacuum process.

In the pre-vacuum process, the vacuum pump 44 is used to depressurize the chamber 42. The inside of the chamber 42 is generally set to the pressure ranging -0.07 MPa to - 0.09 MPa with respect to the atmospheric pressure. In the present embodiment, the internal pressure of the chamber 42 is set to -0.07 MPa.

According to the present embodiment, in the pre-vacuum process, depressurization and raise to the atmospheric pressure are repeated three times. Three depressurizing steps at which the chamber is depressurized to -0.07 MPa are carried out in total. These steps are needed to infiltrate steam into every corner of equipment, which is an object of sterilization, during the succeeding autoclaving process. By depressurizing the chamber 42, high-pressure high-temperature steam permeates the entire equipment that is an object of sterilization.

Thereafter, the autoclave 41 executes the autoclaving process.

In the autoclaving process, the steam feeder 43 is used to feed high-temperature high-pressure steam into the chamber 42. The internal pressure of the chamber 42 is generally set to about +0.2 MPa with respect to the atmospheric pressure.

The temperature condition for the autoclaving process varies depending on the model of the autoclave or the time required for the autoclaving process. In general, the temperature ranges from 115°C to 138°C. In some cases, the temperature may be set to about 142°C.

The time condition for the autoclaving process varies depending on the temperature condition therefor, and is generally set to the range from 3 min. to 60 min. In some cases, the time for the autoclaving process may be set to about 100 min.

Thereafter, in order to dry the autoclaved equipment that is an object of sterilization, the second depressurization process called a dry process at which the chamber 42 is depressurized again is executed after the completion of the autoclaving process.

In this process, the vacuum pump 44 is reused to depressurize the chamber 42. Thus, steam is released from the sterilization chamber in order to facilitate drying of the equipment, which is an object of sterilization, in the sterilization chamber. In this process, the inside of the sterilization chamber is set to the pressure equal to or lower than the pressure of -0.07 MPa attained in the first depressurization process.

According to the present embodiment, in the dry process, depressurization and raise to the atmospheric pressure are repeated three times. Three depressurizing steps are carried out in total.

In the dry process, the first depressurizing step brings the internal pressure of the chamber to -0.08 MPa, the second depressurizing step brings it to -0.085 MPa, and the third depressurizing step brings it to -0.09 MPa. These pressures are lower than the lowest pressure attained in the first depressurization process, and lower than the pressure attained at an immediately preceding depressurizing step within the dry process.

Now, the operation and effect given by the major components of the present embodiment will be described below.

### (Operation)

In order to autoclave the endoscope 2, the waterproof cap 33 is attached to the electric connector 11. Consequently, the communication vent 37 is blocked and the inside of the endoscope 2 is kept watertight and shut out from the outside thereof.

In the pre-vacuum process that is the first depressurization process, the vacuum pump 44 is driven under the control of the control unit 45, and the chamber 42 is depressurized.

At this time, force oriented from the inside of the watertight endoscope 2 to the outside thereof works, as shown in Fig. 6, on the check valve 70 included in the pressure adjustment member 79 formed in the connector unit 10, though it depends on the difference in pressure between the inside and outside of the endoscope 2. The check valve 70 is moved upwards in the drawing against the constraining force exerted by the spring 71 until it abuts on the base body 60. The O ring 76 parts from the seal surface 73. Consequently, the inside and outside of the endoscope 2 communicate with each other through the ventilation grooves 77 and 78. This prevents occurrence of a large difference in pressure between the inside of the endoscope 2 and the chamber 42.

Consequently, an armor member such as a soft and thin sheathing member that sheathes the bending section 16 of the endoscope 2 will not be broken due to a difference in pressure between the inside and outside of the endoscope.

When the internal and external pressures of the endoscope 2 become substantially equal to each other, the check valve 70 is moved with the constraining force exerted by the spring 71. The O ring 76 blocks a gap on the seal surface 73 linking the inside and outside of the endoscope. At this time, the inside of the endoscope 2 is held at substantially the same pressure as the pressure of -0.07 MPa attained in the pre-vacuum process. However, the internal pressure of the endoscope 2 and the pressure attained in the pre-vacuum process have a difference proportional to the constraining force exerted by the spring 71.

Thereafter, the autoclaving process is executed.

The steam feeder 43 feeds high-temperature high-pressure steam into the chamber 42. The chamber 42 is pressurized up to the pressure of +0.02 MPa with respect to the atmospheric pressure. At this time, the check valve 70 shown in Fig. 2 is constrained to move downwards in the drawing. The opening/closing valve 61 is constrained to move upwards in the drawing owing to the constraining force exerted by the spring 62. The O ring 76 and O ring 69 seals the endoscope 2 to keep the inside thereof watertight. The inside of the endoscope 2 is held at -0.07 MPa attained at the pre-vacuum process.

Thereafter, the dry process that is the second depressurization process is executed.

At the first depressurizing step, the vacuum pump 44 is driven under the control of the control unit 45. The chamber 42 is depressurized down to -0.08 MPa. The pressure is lower than the currently retained internal pressure of the endoscope 2 that is -0.07 Pa. Consequently, the check valve 70 opens owing to the same operation as the one exerted in the pre-vacuum process. The gas staying in the endoscope 2 is released to the outside of the endoscope 2. When the internal and external pressures of the endoscope 2 become substantially identical to each other, the check valve 70 is closed. The inside of the endoscope 2 is held at substantially the same pressure as -0.08 Mpa. Thereafter, the pressure in the chamber 42 rises up to the atmospheric pressure.

At the second depressurizing step, the vacuum pump 44 is driven under the control of the control unit 45, and the chamber 42 is depressurized down to -0.085 Mpa.

In the same manner as the aforesaid one, the check valve 70 is opened, and the gas in the endoscope 2 is released to outside. When the internal and external pressures of the endoscope 2 become substantially equal to each other, the check valve 70 is closed. The inside of the endoscope 2 is held at substantially the same pressure as - 0.085 MPa. Thereafter, the pressure in the chamber 42 is raised to the atmospheric pressure.

At the third depressurizing step, the vacuum pump 44 is driven under the control of the control unit 45, and the chamber 42 is depressurized up to the pressure of -0.09 MPa.

In the same manner as the aforesaid one, the check valve 70 is opened, and the gas in the endoscope 2 is released to outside. When the internal and external pressures of the endoscope 2 become substantially equal to each other, the check valve 70 is closed. At this time, the inside of the endoscope 2 is retained substantially at the same pressure as -0.09 MPa.

Humidity having invaded into the endoscope 2 in the autoclaving process is released to the outside of the endoscope 2 when the check valve 70 is opened in the dry process. Consequently, the inside of the endoscope 2 is dried.

In the dry process, the check valve 70 is repeatedly opened, whereby discharge of humidity from the endoscope 2 and drying of the inside of the endoscope 2 are repeatedly carried out.

Thus, autoclaving is completed.

### (Effect)

According to the foregoing first embodiment, the pressure attained at at least one depressurizing step included in the second depressurization process succeeding the autoclaving process is lower than the lowest pressure attained in the first depressurization process preceding the autoclaving process. Consequently, at at least one depressurizing step included in the second depressurization process, the internal pressure of the endoscope 2 is made higher than the external pressure by a certain value or more. Consequently, the check valve 70 is opened reliably. Since the inside of the endoscope 2 is dried through the check valve 70, even if the endoscope 2 is autoclaved, deterioration of the internal parts of the endoscope 2 caused by humidity can be prevented. This results in an autoclaving method according to which even if autoclaving is repeatedly performed, no humidity will be accumulated in the endoscope 2 and the internal parts will not be deteriorated by humidity. Moreover, an autoclave in which the autoclaving method is implemented, and an endoscope system to which the autoclaving method is adapted are provided. Eventually, the reliability of an endoscope can be improved, and the service life thereof can be extended.

Incidentally, the number of depressurizing steps included in the second depressurization process may be only one.

The medical equipment to be autoclaved is not limited to endoscopes. The autoclaving method can be adapted to any medical equipment having a soft armor member as long as the medical equipment includes the pressure adjustment member 79.

Next, a description will be made of the other operations and effects of the first embodiment.

### (Operation)

According to the first embodiment, before the endoscope 2 is used for examination, the waterproof cap 33 is detached from the electric connector 11.

Consequently, the inside and outside of the endoscope 2 communicate with each other through the communication vent 37. The internal depressurized state of the endoscope 2 is discontinued, and the load imposed on the armor of the endoscope 2 is removed. Therefore, the load will not be kept imposed on the armor of the endoscope 2 for a long period of time.

Moreover, according to the first embodiment, the electric connector 11 that is a contact with the signal cable 4 over which external equipment such as the video processor 5 is connected to the endoscope 2 without fail at the time of examination has the communication vent 37. Therefore, a user will not forget to detach the waterproof cap 33. The internal depressurized state of the endoscope 2 will be discontinued reliably. Therefore, the armor will not be broken.

During examination, the inside and outside of the endoscope 2 communicate with each other without fail. The inside of the endoscope 2 is reliably dried. Moreover, since no load is imposed on the soft bendable sheathing rubber of the bending section 16, angulation will not be adversely affected.

Moreover, according to the first embodiment, the pressure adjustment member 79 is formed as an integral part of the endoscope 2. A user will not forget to attach the pressure adjustment member 79 at the time of autoclaving. The damage of the endoscope 2 in the depressurization process can be prevented reliably.

Moreover, according to the first embodiment, after examination is completed, before the endoscope 2 is cleansed, a leakage test is performed in order to check if the endoscope 2 is damaged.

At this time, the waterproof cap 33 is attached to the electric connector 11 in order to block the communication vent 37. A pressurization base 47 connected to a pressurizing source that is not shown is, as shown in Fig. 7, joined to the pressure adjustment member 79.

When the pressurization base 47 is joined to the pressure adjustment member 79, a pressing member 48 thrusts the check valve 70 and opening/closing valve 61 downwards in the drawing while withstanding the constraining force exerted by the spring 62. Consequently, the seal surface 65 parts from the O ring 69. The inside of the endoscope 2 communicates with the inside of the pressurization base 47 through the ventilation grooves 77 and 66. The inside of the endoscope 2 can then be pressurized. A worker immerses the thus pressurized endoscope 2 in water so as to perform a leakage test.

As mentioned above, the connector unit 10 of the endoscope 2 has as one integral part an opening/closing valve (opening/closing valve 61) that is used to pressurize the inside of the endoscope 2 for a leakage test, and a pressure adjustment valve (check valve 70) that is opened when the external pressure of the endoscope 2 is lower than the internal pressure thereof during autoclaving. A special structure or a special procedure is unnecessary for preventing the check valve 70 from opening during a leakage test.

If the waterproof cap 33 is left unattached during a leakage test, the pressurized gas leaks out of the communication vent 37. This allows a user to recognize that he/she forgot to attach the waterproof cap 33. Consequently, the user will not forget to attach a member, with which the communication vent 37 is blocked, before performing autoclaving. The endoscope 2 can be prevented from being damaged with steam having invaded into the endoscope 2.

Moreover, according to the first embodiment, the electric connector 11 having the electric contact pins 38 that must be protected from water during leakage testing, cleansing, or autoclaving includes the communication vent 37. Consequently, when any of the leakage testing, cleansing, or autoclaving is performed, one member serves as both the member for blocking the communication vent 37 and the member for covering the electric connector 11.

Moreover, the endoscope 2 can share the same waterproof cap 33 with an endoscope that is connected to the same external equipment but designed not to be autoclaved.

### (Effect)

According to the first embodiment, a user will not forget to attach the pressure adjustment member 79 at the time of autoclaving, will not forget to attach the waterproof cap 33, which is used to block the communication vent 37, at the time of autoclaving, and will not forget to detach the waterproof cap 33 after the completion of the autoclaving. The user need not work to shield the pressure adjustment member 79 at the time of a leakage test. When autoclaving, cleansing, or leakage testing is performed, the electric connector 11 can be covered at the same time when the communication vent 37 is blocked. According to the first embodiment, an endoscope offering excellent workability can be provided.

Moreover, according to the first embodiment, an endoscope that is highly interchangeable with other endoscopes can be provided because the endoscope can share the same waterproof cap 33 with endoscopes that are connected to the same external equipment but are not designed to be autoclaved.

The communication vent 37 may be formed near the contact of the endoscope 2 via which the endoscope 2 is connected to the light source unit 3 or in the operating unit 8, and the blocking member such as the waterproof cap 33 may be attachable to the communication vent 37 in order to block the communication vent 37. Even when the communication vent 37 is formed near the contact via which the endoscope is connected to the light source unit 3, the blocking member will be reliably removed in order to connect the light source unit 3 to the endoscope 2 at the time of examination. Even when the communication vent 37 is formed in the operating unit 8, since an operator holds and handles the operating unit 8, the blocking member will be removed without fail. The internal depressurized state of the endoscope 2 will be discontinued without fail.

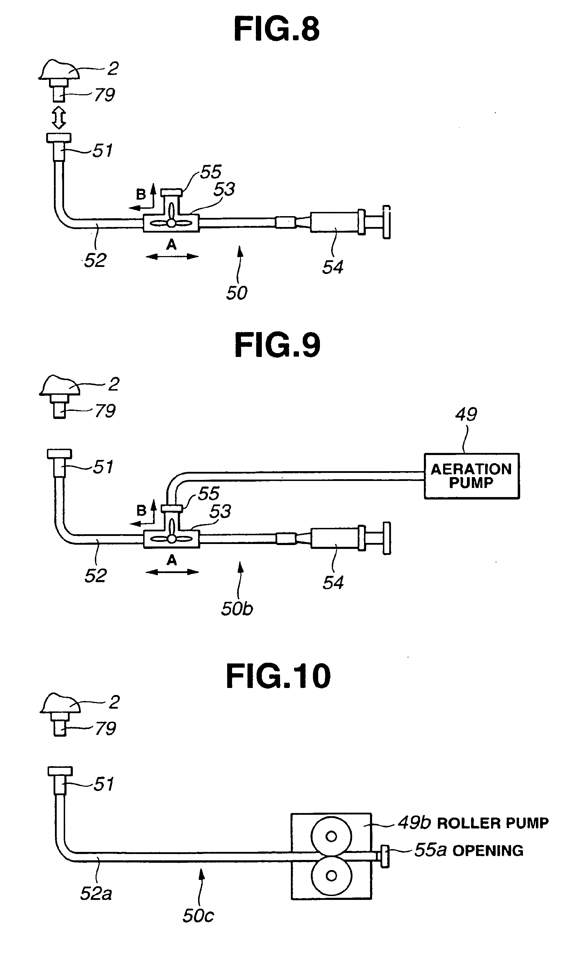

Fig. 8 is an explanatory diagram showing a ventilating means for forcibly ventilating the inside of the endoscope shown in Fig. 1 to Fig. 7.

### (Constituent Features)

As shown in Fig. 8, a ventilating means 50 can be freely detachably attached to the pressure adjustment member 79 of the endoscope 2.

The ventilating means 50 comprises a connection base 51, a tube 52, a three-way cock 53, and a cylinder 54. One opening 55 of the three-way cock 53 opens upon the atmosphere. The connection base 51 has the same structure as the pressurization base 47, and is freely detachably attached to the pressure adjustment member 79. When the connection base 51 is attached to the pressure adjustment member 79, the ventilating means 50 communicates with the inside of the endoscope 2.

### (Operation)

In order to forcibly ventilate the inside of the endoscope 2, the waterproof cap 33 is attached to the electric connector 11 of the endoscope 2. The communication vent 37 is thus blocked.

Thereafter, the connection base 51 is attached to the pressure adjustment member 79, whereby the ventilating means 50 communicates with the inside of the endoscope 2.

Thereafter, the three-way cock 53 is used to link the connection base 51 and syringe 54.

Thereafter, the three-way cock 53 is handled in order to permit the flow indicated with arrow A. Consequently, the gas in the endoscope 2 can be sucked using the syringe 54.

Thereafter, the three-way cock 53 is handled in order to permit the flow indicated with arrow B. Consequently, the connection base 51 and opening 55 communicate with each other. Eventually, the air flows through the opening 55 to the depressurized inside of the endoscope 2, whereby the inside of the endoscope 2 is ventilated.

The above work is repeated in order to ventilate the inside of the endoscope 2.

### (Effect)

Using the ventilating means 50, the inside of the endoscope 2 can be readily ventilated. Humidity accumulated in the endoscope 2 after the completion of autoclaving can be removed.

Fig. 9 is an explanatory diagram showing a first variant of the ventilating means 50 shown in Fig. 8.

As shown in Fig. 9, a ventilating means 50b has a pressurizing source such as an aeration pump 49, which is used for a leakage test, connected to the opening 55. In the case of this structure, after the gas in the endoscope 2 is sucked using the syringe 54, the three-way cock 53 is handled to permit the flow indicated with arrow B. The aeration pump 49 forces outside air to flow into the endoscope 2, whereby the inside of the endoscope 2 is ventilated.

The ventilating means 50b can provide the same advantage as the ventilating means 50 shown in Fig. 9 can. Moreover, since the aeration pump 49 forces outside air to flow into the endoscope 2, the inside of the endoscope 2 can be more efficiently ventilated.

Fig. 10 is an explanatory diagram showing a second variant of the ventilating means 50 shown in Fig. 8.

As shown in Fig. 10, a ventilating means 50c has a roller pump 49b, which can switch forcible feed of a gas and suction thereof, joined to a tube 52a.

In the case of this structure, suction of a gas in the endoscope 2 and forcible feed of outside air to the endoscope 2 are repeated using the roller pump 49, whereby the inside of the endoscope 2 is ventilated.

The ventilating means 50b provides the same advantage as the ventilating means 50a shown in Fig. 9 can.

### (Second Embodiment)

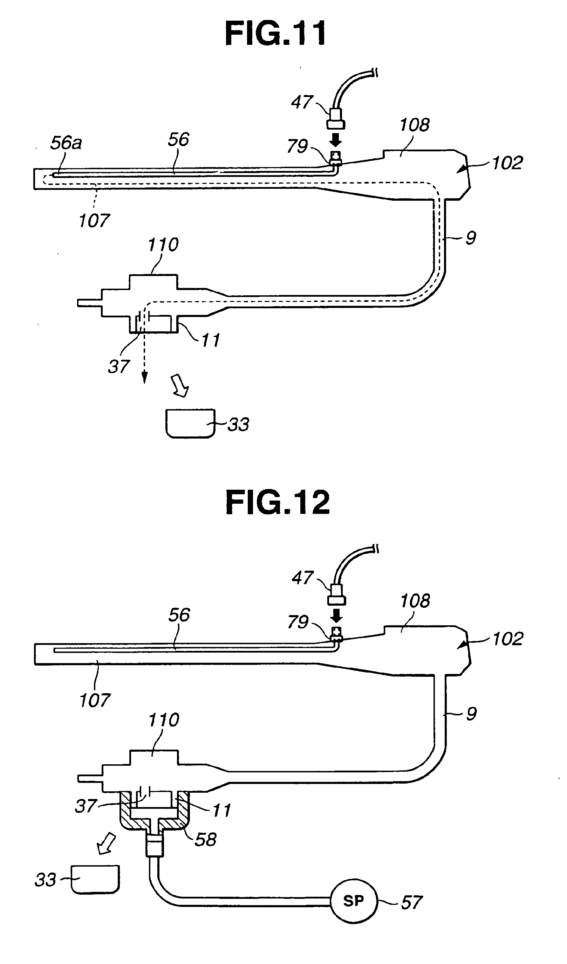

Fig. 11 is an explanatory diagram showing an endoscope and a ventilating means that forcibly ventilates the inside of the endoscope and relates to a second embodiment of the present invention. Referring to Fig. 11, a description will be made with the same reference numerals assigned to components identical to those of the first embodiment shown in Fig. 1 to Fig. 7.

### (Constituent Features)

As shown in Fig. 11, the pressure adjustment member 79 included in the first embodiment is disposed in an operating unit 108 of an endoscope 102. According to the second embodiment, the pressure adjustment member 79 is not disposed in the connector unit 110.

A ventilation tube 56 is inserted through an insertion unit 107 of the endoscope 102. The proximal end of the ventilation tube 56 is joined to the pressure adjustment member 79. The ventilation tube 56 has an opening 56a in the distal section of the insertion unit 107.

### (Operation)

When the inside of the endoscope 102 is forcibly ventilated, the waterproof cap 33 is detached from the electric connector 11 of the endoscope 102 in order to unblock the communication vent 37.

Thereafter, the pressurization base 47 connected to the pressurizing source is joined to the pressure adjustment member 79.

Thereafter, the pressurizing source is activated in order to forcibly feed a gas to the inside of the endoscope 102.

The forcibly fed gas is propagated to the distal section of the insertion unit 107 by way of the pressure adjustment member 79 and ventilation tube 56. Thereafter, the gas passes through the insertion unit 107, operating unit 8, linkage cord 9, and connector unit 110, and is released to the outside of the endoscope 102 through the communication vent 37 in the electric connector 11.

Consequently, the inside of the endoscope 102 is ventilated.

### (Effect)

According to the second embodiment, the opening 56a and communication vent 37 are separated from each other and disposed at one end of the endoscope 102 and the other end thereof respectively. Consequently, the inside of the endoscope 102 can be entirely, readily, and reliably ventilated. Humidity accumulated in the endoscope 102 after the completion of autoclaving can be removed. Eventually, the durability of the endoscope 102 improves.

Incidentally, a suction pump may be substituted for the pressurizing source and connected to the pressure adjustment member 79. While the gas in the endoscope 102 is being sucked, outside air may be taken in through the communication vent 37 in order to ventilate the inside of the endoscope 102.

### (Third Embodiment)

Fig. 12 is an explanatory diagram showing an endoscope, and a ventilating means that forcibly ventilates the inside of the endoscope and relates to a third embodiment of the present invention. Referring to Fig. 12, a description will be made with the same reference numerals assigned to components identical to those of the second embodiment shown in Fig. 11. The description of the identical components will be omitted.

### (Constituent Features)

As shown in Fig. 12, a suction pump 57 has a connection base 58 whose structure is analogous to that of the waterproof cap 33 and which is detachably attached to the electric connector 11.

### (Operation)

In order to forcibly ventilate the inside of the endoscope 102, the connection base 58 is attached to the electric connector 11.

Thereafter, the suction pump 57 is activated in order to suck the gas in the endoscope 102 through the communication vent 37. Moreover, the pressurizing source is activated in order to forcibly feed outside air to the inside of the endoscope 102, whereby the inside of the endoscope 102 is ventilated.

### (Effect)

According to the variant, the inside of the endoscope 102 can be readily ventilated using the suction pump 57. Humidity accumulated in the endoscope 102 after the completion of autoclaving can be removed. Moreover, the durability of the endoscope improves.

Incidentally, a plurality of exhaust ports may be bored in the endoscope 102, and an aeration port may be formed in the middle of the exhaust ports. An aerating source or sucking source may be connected to the aeration port for the purpose of aeration or suction, and an exhaust or gas may be introduced through the exhaust ports. In this case, the time required for ventilation is shortened.

Incidentally, the aeration pump or suction pump included in the ventilating means employed in any of the first to third embodiments, and a means for connecting the aeration pump or suction pump to the endoscope may be incorporated in an autoclave. The autoclave may be designed to autonomously control the actions to be performed for forcibly ventilating the inside of the endoscope after the completion of autoclaving.

### (Fourth Embodiment)

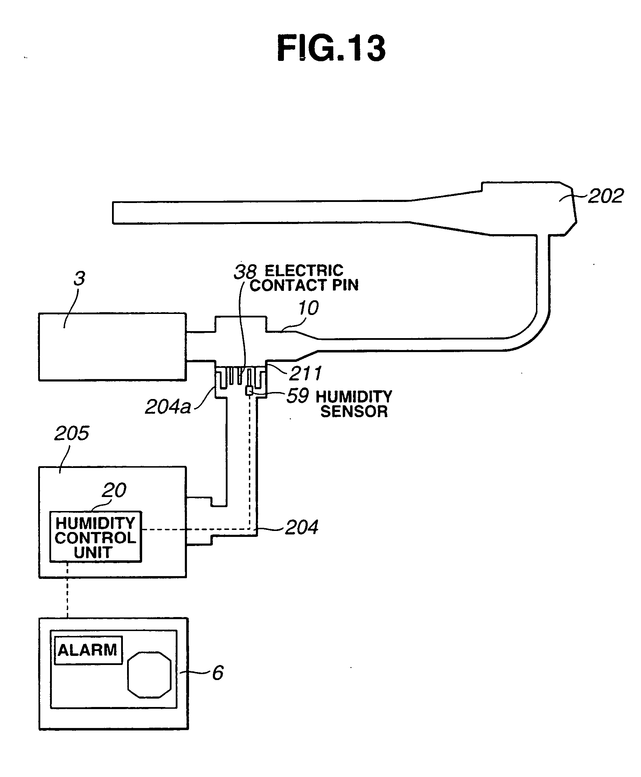

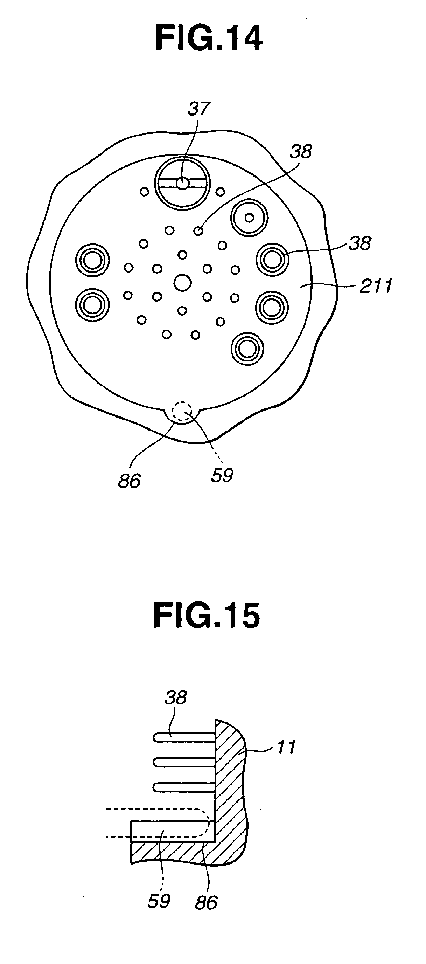

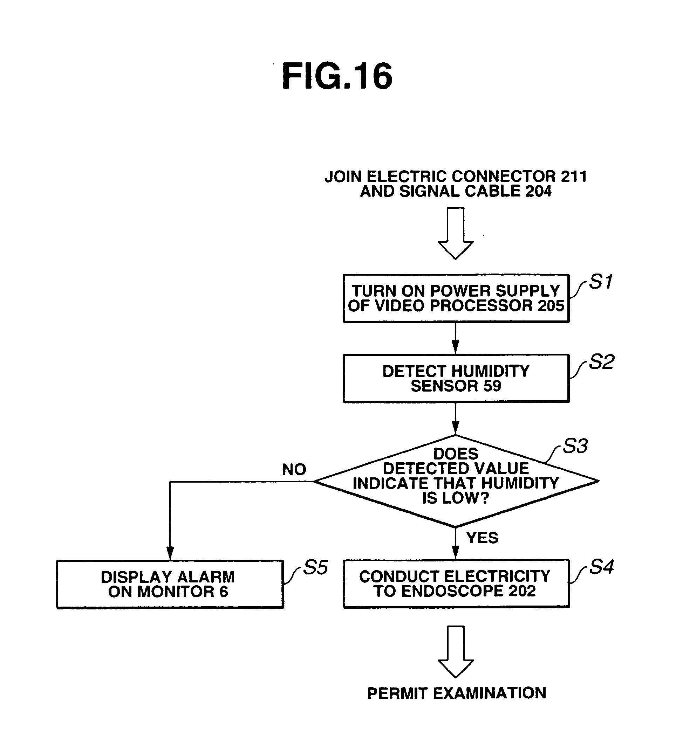

Fig. 13 to Fig. 16 are concerned with a fourth embodiment of the present invention. Fig. 13 is an explanatory diagram showing the overall configuration of an endoscope system. Fig. 14 is a front view of an electric connector. Fig. 15 is a sectional view of the electric connector. Fig. 16 is a flowchart describing a control sequence followed by a humidity control unit. Referring to Fig. 13 to Fig. 16, a description will be made with the same reference numerals assigned to components identical to those of the first embodiment shown in Fig. 1 to Fig. 7. The description of the identical components will be omitted.

### (Constituent Features)

Referring to Fig. 13 to Fig. 15, a description will be made of the configuration for preventing electric conduction from a video processor 205 to an endoscope 202 in case an electric connector 211 of the endoscope 202 has not been dried.

As shown in Fig. 13, a contact 204a of a signal cable 204 to be attached to the electric connector 211 includes a humidity sensor 59 that when the contact 204a is attached to the electric connector 204, projects to the electric connector 211.

The video processor 205 has a humidity control unit 20 connected to the humidity sensor 59.

As shown in Fig. 14 and Fig. 15, the electric connector 211 has a concave portion 86 that is located in a vertically lower part of the electric connector 211 with the signal cable 204 joined to the electric connector 211. The humidity sensor 59 is placed in the concave portion 86.

Referring to Fig. 16, a control sequence to be followed by the humidity control unit 20 included in the video processor 205 will be described below.

As described in Fig. 16, when the electric connector 211 and signal cable 204 are joined at the time of examination, the power supply of the video processor 205 is turned on at step S1. At step S2, the humidity control unit 20 directs the humidity sensor 59 to detect the humidity in the electric connector 211, and then proceeds to step S3.

At step S3, if the humidity control unit 20 judges that the value detected by the humidity sensor 59 is equal to or smaller than a certain value and that the inside of the electric connector 211 is dry enough to conduct electricity, electricity is conducted to the endoscope 202 at step S4. This allows the endoscope 202 to perform normal examination.

At step S3, if the humidity control unit 20 judges that the value detected by the humidity sensor 59 exceeds the certain value and that the inside of the electric connector 211 is wet or the humidity in the electric connector 211 is very high, an alarm is displayed on the monitor 6 at step S5 and electricity is not conducted to the endoscope 202.

### (Operation)

According to the fourth embodiment, if the inside of the electric connector 211 is wet or the humidity in the electric connector 211 is very high, electricity is not conducted to the endoscope 202. The electric contact pins 38 included in the electric connector 211 will not be short-circuited, and an imaging circuit or a remote control circuit incorporated in the endoscope 202 will not be damaged.

Moreover, if the inside of the electric connector 211 is wet or the humidity in the electric connector 211 is very high, an alarm is displayed on the monitor 6 so that a user will be informed of the fact.

### (Effect)

According to the fourth embodiment, there is provided an endoscope system in which even if the electric connector 211 is wet because of cleansing or autoclaving, the endoscope 202 will not be damaged.

### (Fifth Embodiment)

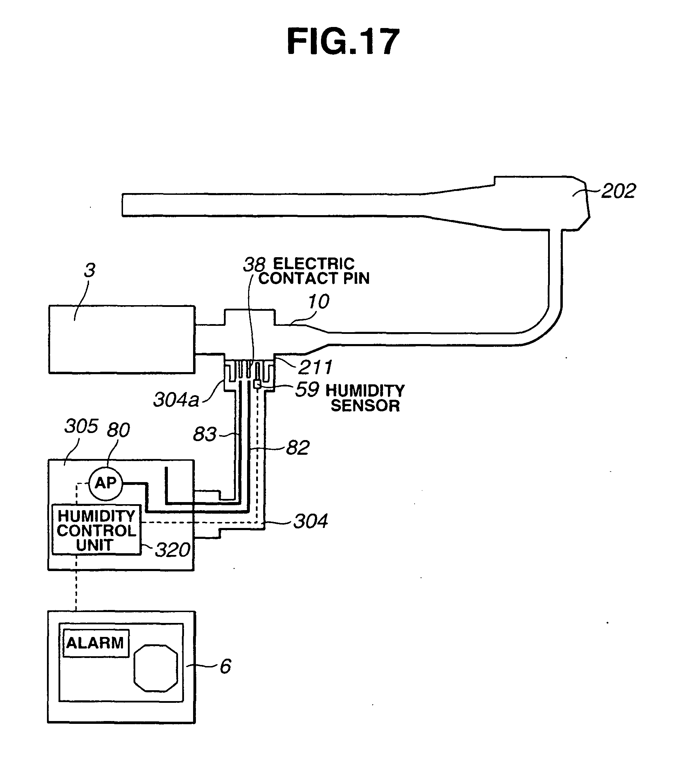

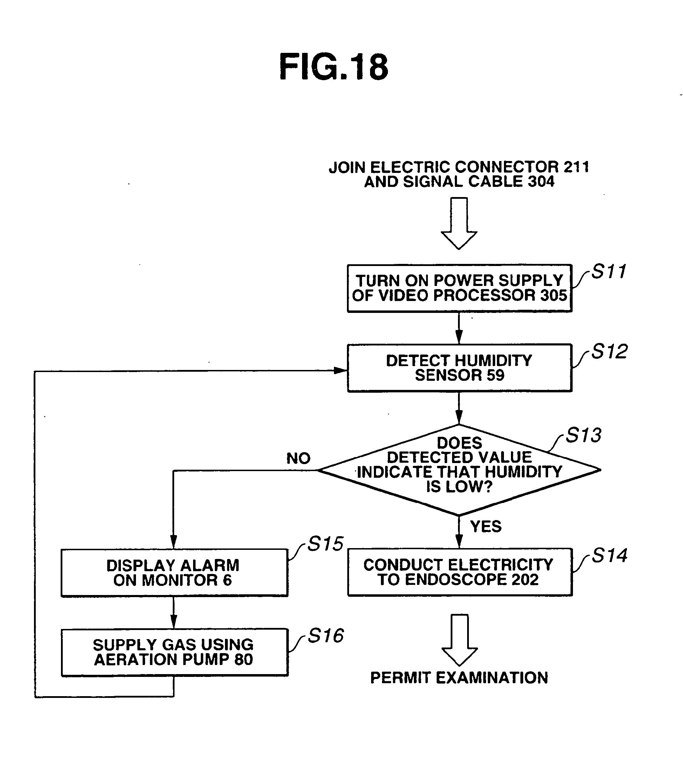

Fig. 17 and Fig. 18 are concerned with a fifth embodiment of the present invention. Fig. 17 is an explanatory diagram showing the overall configuration of an endoscope system. Fig. 18 is a flowchart describing a control sequence to be followed by a humidity control unit. Referring to Fig. 17 and Fig. 18, a description will be made with the same reference numerals assigned to components identical to those of the fourth embodiment shown in Fig. 13 to Fig. 16. The description of the identical components will be omitted.

### (Constituent Features)

As shown in Fig. 17, a contact 304a of a signal cable 304 includes the humidity sensor 59. The humidity sensor 59 is connected to a humidity control unit 320 included in a video processor 305. The video processor 305 includes an aeration pump 80 connected to the humidity control unit 320. An aeration tube 82 coupled to the aeration pump 80 and an exhaust tube 83 are contained in the signal cable 304. The aeration tube 82 and exhaust tube 83 open onto the contact 304a, which is attached to the electric connector 211, at one ends thereof. The other end of the exhaust tube 83 opens upon the atmosphere.

Referring to Fig. 18, a control sequence to be followed by the humidity control unit 320 included in the video processor 305 will be described below.

As described in Fig. 18, a control sequence from step S11 to S15 followed by the humidity control unit 320 is identical to a control sequence from step S1 to S5 followed by the humidity control unit 220 and described in Fig. 16.

A difference of the fifth embodiment from the fourth embodiment lies in that: at step S15, an alarm is displayed on the monitor 6 and electricity is not conducted to the endoscope 202; and at step S16, the humidity control unit 320 directs the aeration pump 80 to perform aeration and control is returned to step S12.

Owing to the sequence followed by the humidity control unit 320, the humidity sensor 59 detects humidity at regular intervals, for example, at intervals of 30 sec., and the aeration pump 80 supplies a gas until the humidity becomes equal to or smaller than the certain value. When the humidity becomes equal to or smaller than the certain value, the aeration pump 80 stops supplying the gas, and the video processor 305 starts conducting electricity to the endoscope 302 and terminates the display of the alarm on the monitor 6.

### (Operation)

According to the fifth embodiment, a gas forcibly supplied by the aeration pump 80 is introduced into the electric connector 211 by way of the aeration tube 82, and released to the atmosphere by way of the exhaust tube 83. Consequently, the inside of the electric connector 211 is dried.

### (Effect)

The fifth embodiment provides the same advantage as the fourth embodiment does. In addition, the electric connector 211 is dried automatically.

The humidity sensor 59 may be included in the electric connector 211 of the endoscope 202 but not in the signal cable 304 so that when the electric connector 211 and signal cable 304 are joined, the endoscope 202 and the humidity control unit 20 in the video processor 305 will be connected to each other.

Moreover, when the endoscope 202 has an electric contact via which the endoscope is connected to the light source unit 3, the humidity sensor 59 may be included in the light source unit 3. When the light source unit 3 and video processor 305 are integrated into one unit, the humidity sensor 59 may be included in the unit.

### (Sixth Embodiment)

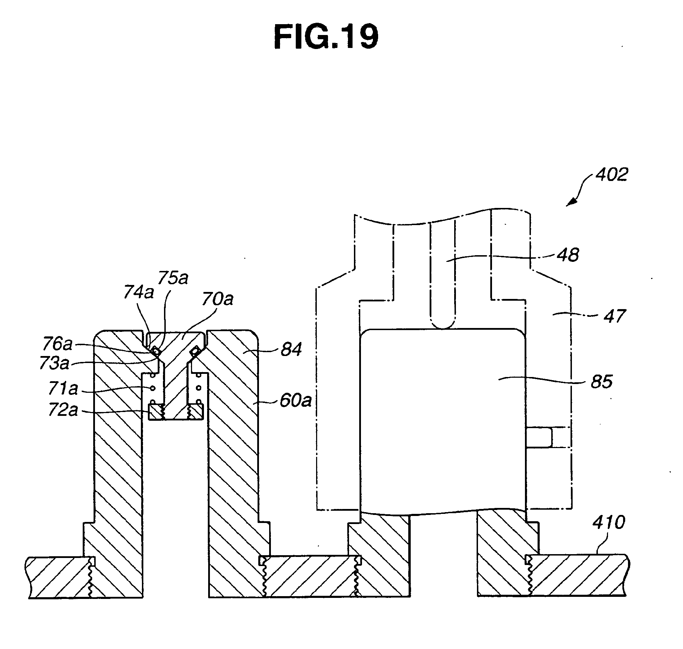

Fig. 19 is a sectional view showing a major portion of an endoscope relating to a sixth embodiment of the present invention. The components of an endoscope system other than the one shown in Fig. 19 are identical to those of the endoscope system to which the first embodiment shown in Fig. 1 to Fig. 7 is adapted.

In the endoscope 402 according to the sixth embodiment, an opening/closing valve 85 via which the inside of an endoscope 302 is pressurized for a leakage test, and a pressure adjustment member 84 that opens when the external pressure of the endoscope 302 is lower than the internal pressure thereof during autoclaving are mutually independently included in a connector unit 410.

The pressure adjustment member 84 comprises a base body 60a, a check valve 70a, a spring 71a, a spring bracket 72a, an O ring groove 75a, and an O ring 76a. The check valve 70a is movably engaged with the base body 60a, and the spring 71a constrains the check valve 70a to move downwards in the drawing. The spring bracket 72a prevents the spring 71a from coming off. The base body 60a has a seal surface 73a.

The check valve 70a has an abutment surface 74a that abuts on the seal surface 73a. The O ring groove 75a is formed in the abutment surface 74a, and the O ring 76a is fitted in the O ring groove 75a.

Assuming that the pressure causing the check valve 70a to open is P, the pressure used for pressurization performed at the time of a leakage test is P1, and the pressure applied from the outside of the endoscope 302 to the inside thereof in a depressurization process during autoclaving is P2, a degree of force exerted by the spring 71a is determined so that P1<P<P2 will be established.

According to the present embodiment, P1 is set to 0.05 MPa, P2 is set to 0.07 MPa, and P is set to 0.06 MPa.

### (Operation)

According to the sixth embodiment, upon a leakage test, even when the endoscope is pressurized through the opening/closing valve 85 to which the pressurization base 47 is attached, the check valve 70a is not opened. Therefore, a leakage test can be carried out.

Moreover, according to the sixth embodiment, when the endoscope is depressurized down to -0.07 MPa in an autoclaving or depressurization process, the check valve 70a opens. Therefore, occurrence of a large difference in pressure between the inside and outside of the endoscope 302 can be prevented. Eventually, causing damage to the armor of the endoscope 302 can be prevented.

### (Effect)

According to the sixth embodiment, a special configuration or work for keeping the check valve 70a closed during a leakage test is unnecessary. This results in an endoscope system offering excellent workability and enjoying a simple structure.

The embodiments of the present invention have been described so far. However, the present invention is not limited to the embodiments. Needless to say, the present invention can be modified in various manners without a departure from the spirit thereof.

### Industrial Applicability

As described so far, according to the present invention, even when autoclaving is performed, deterioration of the internal parts of medical equipment deriving from humidity can be prevented. Consequently, the reliability of the medical equipment is improved, and the service life thereof is extended.

## Claims

1. A medical equipment autoclaving system comprising:
a medical device (2) comprising:
- a communication vent (37) through which the inside of the medical device (2) and the outside thereof communicate with each other, and
- a pressure adjusting means (79) that includes a check valve (70) which opens only when the pressure in the inside of the medical device (2) which communicates with the outside thereof through the communication vent (37) gets higher than the pressure in the outside thereof by a certain value, and
an autoclave (41) that sterilizes the medical device (2), wherein the autoclave is designed to execute:
- a first depressurization process including a step of depressurizing the inside of a chamber (42) included in the autoclave (41);
- an autoclaving process which succeeds the first depressurization process and in which the chamber (42) is pressurized; and
- a second depressurization process succeeding the autoclaving process and including a step of depressurizing the chamber (42), wherein:
the pressure attained at at least one depressurizing step included in the second depressurization process is lower than the lowest pressure attained in the first depressurization process.

2. The medical equipment autoclaving system according to claim 1, wherein the second depressurization process includes a plurality of depressurizing steps and a plurality of pressurizing steps, and the pressure attained at at least one of the depressurizing steps is lower than the pressure attained at other depressurizing step preceding the depressurizing step.

3. The medical equipment autoclaving system according to claim 1, wherein the medical device (2) includes an armor member designed to shut out the inside of the medical device (2) from the outside thereof and made of a material having softness.

4. The medical equipment autoclaving system according to claim 3, wherein the medical device (2) is an endoscope having a bending section (16) that is formed adjacently to the distal section of an insertion unit (7), which is capable of being inserted into an object, so that it can be bent, and an armor member used to sheathe the bending section (16) and made of a material having softness.

5. The medical equipment autoclaving system according to claim 1, wherein the medical device (2) has the communication vent (37) formed so that the communication vent (37) can be forcibly unblocked after the completion of the second depressurization process.

6. A medical equipment autoclaving method comprising:
- a first depressurization process including a step of depressurizing a chamber (42) included in an autoclave (41) in which a medical device (2) is stored;
- an autoclaving process which succeeds the first depressurization process and in which the chamber (42) is pressurized; and
- a second depressurization process succeeding the autoclaving process and including a step of depressurizing the chamber (42), wherein:
the pressure attained at at least one depressurizing step included in the second depressurization process to be executed by the autoclave (41) is lower than the lowest pressure attained in the first depressurization process, and wherein:
the medical device (2) includes
a communication vent (37) through which the inside of the medical device (2) and the outside thereof communicate with each other, and
a pressure adjusting means (79) having a check valve (70) that opens only when the pressure in the inside of the medical device (2), which communicates with the outside thereof through the communication vent (37), gets higher than the pressure in the outside thereof by a certain value.

7. The medical equipment autoclaving method according to claim 6, wherein
the first and second depressurization processes include a step of forcibly unblocking the communication vent (37) so as to permit the inside of the medical device (2) to communicate with the outside thereof.

8. The medical equipment autoclaving method according to claim 7, wherein the medical device (2) includes an armor member designed to shut out the inside of the medical device (2) from the outside thereof and made of a material having softness.

9. The medical equipment autoclaving method according to claim 8, wherein the medical device (2) is an endoscope having a bending section (16) that is formed adjacently to the distal section of an insertion unit (7), which is capable of being inserted into an object, so that it can be bent, and an armor member used to sheathe the bending section (16) and made of a material having softness.

10. The medical equipment autoclaving method according to claim 7, wherein the medical device (2) is formed so that the communication vent (37) can be forcibly unblocked to permit the inside of the medical device (2) to communicate with the outside thereof after the completion of the second depressurization process.

11. The medical equipment autoclaving method according to claim 6, wherein the second depressurization process includes a plurality of depressurizing steps and a plurality of pressurizing steps, and the pressure attained at at least one of the depressurizing steps is lower than the pressure attained at other depressurizing step preceding the depressurizing step.

## Patentansprüche

1. System zur Autoklavbehandlung medizinischer Geräte, das umfasst:
ein medizinisches Gerät (2), das umfasst:
- eine Verbindungsöffnung (37), über die der Innenraum des medizinischen Gerätes (2) und seine Außenumgebung miteinander in Verbindung stehen, und
- eine Druckreguliereinrichtung (79), die ein Rückschlagventil (70) enthält, das sich nur öffnet, wenn der Druck im Innenraum des medizinischen Gerätes (2), der über die Verbindungsöffnung (37) mit seiner Außenumgebung in Verbindung steht, den Druck in seiner Außenumgebung um einen bestimmten Wert übersteigt, und
einen Autoklaven (41), mit dem das medizinische Gerät (2) sterilisiert wird, wobei der Autoklav dazu dient, folgende Vorgänge auszuführen:
- einen ersten Druckverringerungsvorgang, der einen Schritt des Verringerns des Drucks im Innenraum einer Kammer (42) einschließt, die in dem Autoklaven (41) enthalten ist;
- einen Autoklavbehandlungsvorgang, der auf den ersten Druckverringerungsvorgang folgt und in dem der Druck in der Kammer (42) erhöht wird; und
- einen zweiten Druckverringerungsvorgang, der auf den Autoklavbehandlungsvorgang folgt und einen Schritt des Verringerns des Drucks in der Kammer (42) einschließt, wobei:
der Druck, der in wenigstens einem in dem zweiten Druckverringerungsvorgang eingeschlossenen Druckverringerungsschritt erreicht wird, niedriger ist als der niedrigste in dem ersten Druckverringerungsvorgang erreichte Druck.

2. System zur Autoklavbehandlung medizinischer Geräte nach Anspruch 1, wobei der zweite Druckverringerungsvorgang eine Vielzahl von Druckverringerungsschritten und eine Vielzahl von Druckerhöhungsschritten einschließt und der Druck, der in wenigstens einem der Druckverringerungsschritte erreicht wird, niedriger ist als der Druck, der in dem anderen, dem Druckverringerungsschritt vorangehenden Druckverringerungsschritt erreicht wird.

3. System zur Autoklavbehandlung medizinischer Geräte nach Anspruch 1, wobei das medizinische Gerät (2) ein Schutzelement enthält, das dazu dient, den Innenraum des medizinischen Gerätes (2) gegenüber seiner Außenumgebung abzusperren, und das aus einem weichen Material besteht.

4. System zur Autoklavbehandlung medizinischer Geräte nach Anspruch 3, wobei das medizinische Gerät (2) ein Endoskop ist, das einen Biegeabschnitt (16), der angrenzend an den distalen Abschnitt einer Einführeinheit (7), die in ein Objekt eingeführt werden kann, so ausgebildet ist, dass er gebogen werden kann, und ein Schutzelement aufweist, das verwendet wird, um den Biegeabschnitt (16) zu umhüllen, und das aus einem weichen Material besteht.

5. System zur Autoklavbehandlung medizinischer Geräte nach Anspruch 1, wobei die Verbindungsöffnung (37) des medizinischen Gerätes (2) so ausgebildet ist, dass die Verbindungsöffnung (37) nach dem Abschluss des zweiten Druckverringerungsvorgangs zwangsweise freigegeben werden kann.

6. Verfahren zur Autoklavbehandlung medizinischer Geräte, das umfasst:
- einen ersten Druckverringerungsvorgang, der einen Schritt des Verringerns des Drucks in einer Kammer (42) einschließt, die in einem Autoklaven (41) enthalten ist, in dem ein medizinisches Gerät (2) aufbewahrt wird;
- einen Autoklavbehandlungsvorgang, der auf den ersten Druckverringerungsvorgang folgt und in dem der Druck in der Kammer (42) erhöht wird; und
- einen zweiten Druckverringerungsvorgang, der auf den Autoklavbehandlungsvorgang folgt und einen Schritt einschließt, in dem der Druck in der Kammer (42) verringert wird, wobei:
der Druck, der in wenigstens einem Druckverringerungsschritt erreicht wird, der in dem durch den Autoklaven (41) auszuführenden zweiten Druckverringerungsvorgang eingeschlossen ist, niedriger ist als der niedrigste in dem ersten Druckverringerungsvorgang erreichte Druck, und wobei:
das medizinische Gerät (2) enthält:
eine Verbindungsöffnung (37), über die der Innenraum des medizinischen Gerätes (2) und seine Außenumgebung miteinander in Verbindung stehen, und eine Druckreguliereinrichtung (79), die ein Rückschlagventil (70) aufweist, das sich nur öffnet, wenn der Druck im Innenraum des medizinischen Gerätes, der über die Verbindungsöffnung (37) mit seiner Außenumgebung in Verbindung steht, den Druck in seiner Außenumgebung um einen bestimmten Wert übersteigt.

7. Verfahren zur Autoklavbehandlung medizinischer Geräte nach Anspruch 6, wobei der erste und der zweite Druckverringerungsvorgang einen Schritt einschließen, in dem die Verbindungsöffnung (37) zwangsweise freigegeben wird, um zuzulassen, dass der Innenraum des medizinischen Gerätes (2) mit seiner Außenumgebung in Verbindung steht.

8. Verfahren zur Autoklavbehandlung medizinischer Geräte nach Anspruch 7, wobei das medizinische Gerät (2) ein Schutzelement enthält, das dazu dient, den Innenraum des medizinischen Gerätes (2) gegenüber seiner Außenumgebung abzusperren, und das aus einem weichen Material besteht.

9. Verfahren zur Autoklavbehandlung medizinischer Geräte nach Anspruch 8, wobei das medizinische Gerät (2) ein Endoskop ist, das einen Biegeabschnitt (16), der angrenzend an den distalen Abschnitt einer Einführeinheit (7), die in ein Objekt eingeführt werden kann, so ausgebildet ist, dass er gebogen werden kann, und ein Schutzelement aufweist, das verwendet wird, um den Biegeabschnitt (16) zu umhüllen, und das aus einem weichen Material besteht.

10. Verfahren zur Autoklavbehandlung medizinischer Geräte nach Anspruch 7, wobei das medizinische Gerät (2) so ausgebildet ist, dass die Verbindungsöffnung (37) zwangsweise freigegeben werden kann, um zuzulassen, dass der Innenraum des medizinischen Gerätes (2) nach dem Abschluss des zweiten Druckverringerungsvorgangs mit seiner Außenumgebung in Verbindung steht.

11. Verfahren zur Autoklavbehandlung medizinischer Geräte nach Anspruch 6, wobei der zweite Druckverringerungsvorgang eine Vielzahl von Druckverringerungsschritten und eine Vielzahl von Druckerhöhungsschritten einschließt und der Druck, der in wenigstens einem der Druckverringerungsschritte erreicht wird, niedriger ist als der Druck, der in dem anderen, dem Druckverringerungsschritt vorangehenden Druckverringerungsschritt erreicht wird.

## Revendications

1. Système d'autoclavage d'équipement médical comprenant :
un dispositif médical (2) comprenant:
- un évent de communication (37) à travers lequel l'intérieur du dispositif médical (2) et l'extérieur de celui-ci communiquent l'un avec l'autre, et
- un moyen d'ajustement de pression (79) qui inclut un clapet anti-retour (70) qui ne s'ouvre que lorsque la pression dans l'intérieur du dispositif médical (2) qui communique avec l'extérieur de celui-ci par l'évent de communication (37) devient supérieure à la pression dans l'extérieur de celui-ci d'une certaine valeur, et
un autoclave (41) qui stérilise le dispositif médical (2), dans lequel l'autoclave est conçu pour exécuter:
- un premier processus de dépressurisation incluant une étape de dépressurisation de l'intérieur d'une chambre (42) incluse dans l'autoclave (41);
- un processus d'autoclavage qui fait suite au premier processus de dépressurisation et dans lequel la chambre (42) est pressurisée; et
- un deuxième processus de dépressurisation faisant suite au processus d'autoclavage et incluant une étape de dépressurisation de la chambre (42), dans lequel :
la pression atteinte lors d'au moins une étape de dépressurisation incluse dans le deuxième processus de dépressurisation est plus basse que la pression la plus basse atteinte dans le premier processus de dépressurisation.

2. Système d'autoclavage d'équipement médical selon la revendication 1, dans lequel le deuxième processus de dépressurisation inclut une pluralité d'étapes de dépressurisation et une pluralité d'étapes de pressurisation, et la pression atteinte lors d'au moins une étape de dépressurisation est plus basse que la pression atteinte lors d'une autre étape de dépressurisation précédant l'étape de dépressurisation.

3. Système d'autoclavage d'équipement médical selon la revendication 1, dans lequel le dispositif médical (2) inclut un élément de blindage conçu pour isoler l'intérieur du dispositif médical (2) de l'extérieur de celui-ci et constitué d'un matériau ayant une souplesse.

4. Système d'autoclavage d'équipement médical selon la revendication 3, dans lequel le dispositif médical (2) est un endoscope ayant une section pouvant se courber (16) qui est formée de façon adjacente à la section distale d'une unité d'insertion (7), qui est apte à être insérée dans un objet, de sorte qu'elle peut être courbée, et un élément de blindage utilisé pour gainer la section pouvant se courber (16) et constitué d'un matériau ayant une souplesse.

5. Système d'autoclavage d'équipement médical selon la revendication 1, dans lequel le dispositif médical (2) a l'évent de communication (37) formé de façon à ce que l'évent de communication (37) puisse être débloqué de force après la fin du deuxième processus de dépressurisation.

6. Procédé d'autoclavage d'équipement médical comprenant:
- un premier processus de dépressurisation incluant une étape de dépressurisation d'une chambre (42) incluse dans un autoclave (41) dans lequel un dispositif médical (2) est stocké;
- un processus d'autoclavage qui fait suite au premier processus de dépressurisation et dans lequel la chambre (42) est pressurisée; et
- un deuxième processus de dépressurisation faisant suite au processus d'autoclavage et incluant une étape de dépressurisation de la chambre (42), dans lequel:
la pression atteinte lors d'au moins une étape de dépressurisation incluse dans le deuxième processus de dépressurisation devant être exécuté par l'autoclave (41) est plus basse que la pression la plus basse atteinte dans le premier processus de dépressurisation, et dans lequel:
le dispositif médical (2) inclut
un évent de communication (37) à travers lequel l'intérieur du dispositif médical (2) et l'extérieur de celui-ci communiquent l'un avec l'autre, et
un moyen d'ajustement de pression (79) ayant un clapet anti-retour (70) qui ne s'ouvre que lorsque la pression dans l'intérieur du dispositif médical (2), qui communique avec l'extérieur de celui-ci par l'évent de communication (37), devient supérieure à la pression dans l'extérieur de celui-ci d'une certaine valeur.

7. Procédé d'autoclavage d'équipement médical selon la revendication 6, dans lequel le premier et le deuxième processus de dépressurisation incluent une étape de déblocage de force de l'évent de communication (37) de façon à permettre que l'intérieur du dispositif médical (2) communique avec l'extérieur de celui-ci.

8. Procédé d'autoclavage d'équipement médical selon la revendication 7, dans lequel le dispositif médical (2) inclut un élément de blindage conçu pour isoler l'intérieur du dispositif médical (2) de l'extérieur de celui-ci et constitué d'un matériau ayant une souplesse.

9. Procédé d'autoclavage d'équipement médical selon la revendication 8, dans lequel le dispositif médical (2) est un endoscope ayant une section pouvant se courber (16) qui est formée de façon adjacente à la section distale d'une unité d'insertion (7), qui est apte à être insérée dans un objet, de sorte qu'elle peut être courbée, et un élément de blindage utilisé pour gainer la section pouvant se courber (16) et constitué d'un matériau ayant une souplesse.

10. Procédé d'autoclavage d'équipement médical selon la revendication 7, dans lequel le dispositif médical (2) est formé de façon à ce que l'évent de communication (37) puisse être débloqué de force de façon à permettre que l'intérieur du dispositif médical (2) communique avec l'extérieur de celui-ci après la fin du deuxième processus de dépressurisation.

11. Procédé d'autoclavage d'équipement médical selon la revendication 6, dans lequel le deuxième processus de dépressurisation inclut une pluralité d'étapes de dépressurisation et une pluralité d'étapes de pressurisation, et la pression atteinte lors d'au moins une des étapes de dépressurisation est plus basse que la pression atteinte lors d'une autre étape de dépressurisation précédant l'étape de dépressurisation.
